# EUROPÄISCHE PATENTANMELDUNG

(11) **EP 0 630 639 A1**
(43) Veröffentlichungstag der Anmeldung: **28.12.1994**
(21) Anmeldenummer: 94108985.6
(22) Anmeldetag: 12.06.1994
(51) Int. Cl.: A61K 6/06

(54) **Dentalkeramische Restauration mit einem mehrschichtigen Aufbau**

(30) Priorität: 25.06.1993 DE 4321100; 21.09.1993 DE 4332025; 09.10.1993 DE 4334493
(71) Anmelder: Vita Zahnfabrik H. Rauter GmbH & Co KG, D-79713 Bad Säckingen (DE)
(72) Erfinder: Thiel, Norbert, Dr., D-79713 Harpolingen (DE); Weber, Susanne, D-79739 Schwörstadt (DE)
(74) Vertreter: Meyers, Hans-Wilhelm, Dr.Dipl.-Chem.

(57) **Zusammenfassung**

Dentalkeramische Restauration mit einem mehrschichtigen Aufbau auf einem Kern, erhältlich durch Aufbringen einer Opaker-Schicht der Zusammensetzung
50 bis 60 Gew.-% SiO₂, 4 bis 10 Gew.-% Na₂O, 0 bis 2,5 Gew.-% CaO, 8 bis 14 Gew.-% K₂O, 10 bis 20 Gew.-% Al₂O₃, 0 bis 6 Gew.-% B₂O₃, 5 bis 12 Gew.-% TiO₂ sowie Pigmenten, vorzugsweise auf Oxidbasis, auf einen Kern, gefolgt von Brennen dieser Anordnung,
danach Aufbringen einer Dentin-/Schmelzschicht der Zusammensetzung 50 bis 60 Gew.-% SiO₂, 4 bis 10 Gew.-% Na₂O, 0 bis 2,5 Gew.-% CaO, 8 bis 14 Gew.-% K₂O, 10 bis 20 Gew.-% Al₂O₃, 2 bis 10 Gew.-% B₂O₃, insbesondere 4 bis 8 Gew.-% B₂O₃ sowie Pigmenten, vorzugsweise auf Oxidbasis, gefolgt von einem Brennen der Anordnung.

## Beschreibung

Gegenstand der vorliegenden Erfindung ist eine dentalkeramische Restauration mit einem mehrschichtigen Aufbau auf einem Kern sowie die Verwendung von Zwischenprodukten zur Herstellung einer solchen dentalkeramischen Restauration.

Bei der dentalkeramischen Restauration handelt es sich um festsitzenden oder abnehmbaren metallischen Zahnersatz, der aus ästhetischen und funktionellen Gründen mit einer zahnfarbenen Keramik dauerhaft verblendet wird. Um eine möglichst große Ähnlichkeit mit den natürlichen Zähnen zu erreichen, werden auf ein Metallgerüst in mehreren Stufen keramische Masseschichten aufgebracht, die nach jedem Arbeitsgang gebrannt werden. Hierdurch wird ein dem natürlichen Zahn sehr ähnliches Aussehen der Restauration erreicht.

Die aufzubringenden Keramikmassen bestehen im allgemeinen aus Oxiden verschiedener Metalle, wobei den größten Anteil im allgemeinen die Oxide des Siliziums einnehmen.

Nach dem bisherigen Stand der Technik unterscheidet man beim Aufbau der Metallkeramik die folgenden Keramikschichten, die nach der Legierung aufgebracht werden: Zunächst wird auf die Legierung eine sogenannte Grundmasse (Opaker) aufgebracht. Mit dieser Masse wird erreicht, daß das relativ dunkel gefärbte Metallgerüst der Metallkeramik nicht durchscheint. Danach wird zunächst Dentinmasse mit einer dem natürlichen Zahnbein (Dentin) entsprechenden Semitransparenz aufgetragen, dann wird die relativ transparente Schmelzmasse aufgetragen, und zuletzt können noch verschiedene Effektmassen verwendet werden, um eine individuelle Anpassung an die natürlichen Zähne vornehmen zu können. Zwischen dem Aufbringen der jeweiligen Massen erfolgen nach Bedarf verschiedene Brennvorgänge.

Als Legierung werden zum Beispiel Metallegierungen des Goldes und des Platins verwendet, die silbrig bis hellgelb sind. Um eine ausreichende Haftung zwischen dem Metallgerüst und der Keramikmasse zu erreichen, werden die Legierungen bei einer Temperatur von etwa 950°C vor dem Aufbringen der keramischen Massen geglüht. Dabei entsteht durch Zusatz anderer Elemente eine Oxidschicht auf dem Metall, die eine bessere Haftung zwischen Metall und Keramik ermöglicht.

Neben diesen sehr teuren Legierungen sind inzwischen auch Legierungen auf dem Markt erhältlich, die eine goldgelbe Färbung besitzen und früher als Vollgußlegierungen für metallischen und reinmetallischen Zahnersatz verwendet wurden. Der Kupferanteil dieser Legierungen kann bei etwa 3 bis 7% liegen, kann jedoch auch erheblich höher sein. Derartige Legierungen müssen ebenfalls bei einer Temperatur von ca. 800°C oxidgebrannt werden, um die Haftung mit den keramischen Massen zu verbessern.

Bei der Verwendung dieser Legierungen für eine Metallkeramik tritt jedoch das Problem auf, daß sich die Keramiken nach dem Brand auf der Außenseite schwarz oder grün verfärben. So können beispielsweise schwarze oder grüne Ränder entstehen, die auffällig sind und daher für Zahnersatz nicht erwünscht sein können.

Auch die zur Herstellung von Restaurationen im Stand der Technik bekannten Keramikmassen genügen oft ästhetischen Ansprüchen nicht, da sie teilweise zu Verfärbungen neigen oder eine ungenügende Transluzenz oder Transparenz der dentalkeramischen Restaurationen zu beobachten ist. Dies führt zu einem nicht natürlichen Aussehen des Zahnersatzes. Dies ist sowohl aus ärztlicher wie auch aus kosmetischer Sicht unbefriedigend. So weisen die in der EP 0 475 528 oder P 40 31 168 beschriebenen dentalkeramischen Massen keine ästhetisch befriedigenden Ergebnisse auf.

Das der Erfindung zugrundeliegende technische Problem ist mithin die Bereitstellung von dentalkeramischen Restaurationen, die die genannten Nachteile des Standes der Technik vermeiden.

Dieses technische Problem wird gelöst durch eine dentalkeramische Restauration mit einem mehrschichtigen Aufbau auf einem Kern, erhältlich durch Aufbringen einer Opaker-Schicht der Zusammensetzung 50 bis 60 Gew.-% SiO₂, 4 bis 10 Gew.-% Na₂O, 0 bis 2,5 Gew.-% CaO, 8 bis 14 Gew.-% K₂O, 10 bis 20 Gew.-% Al₂O₃, 0 bis 6 Gew.-% B₂O₃, 5 bis 12 Gew.-% TiO₂ sowie Pigmenten, vorzugsweise auf Oxidbasis, auf einen Kern, gefolgt von einem Brennen dieser Anordnung, danach Aufbringen einer Dentin-/Schmelzschicht der Zusammensetzung 50 bis 60 Gew.-% SiO₂, 4 bis 10 Gew.-% Na₂O, 0 bis 2,5 Gew.-% CaO, 8 bis 14 Gew.-% K₂O, 10 bis 20 Gew.-% Al₂O₃, 2 bis 10 Gew.-% B₂O₃, insbesondere 4 bis 8 Gew.-% B₂O₃ sowie Pigmente, vorzugsweise auf Oxidbasis, gefolgt von einem Brennen der Anordnung.

Unter dem Begriff dentalkeramische Restauration werden insbesondere Kronen, Brücken, Teil- oder Totalprothesen etc. sowie teilkeramische Restaurationen verstanden. Die Restauration kann dabei entweder einen Metall- oder Vollkeramikkern aufweisen.

In einer bevorzugten Ausführungsform wird als erste Schicht auf den Kern eine Bonder-Schicht der nachstehenden Zusammensetzung 50 bis 60 Gew.-% SiO₂, 4 bis 10 Gew.-% Na₂O, 0 bis 2,5 Gew.-% CaO, 8 bis 14 Gew.-% K₂O, 10 bis 20 Gew.-% Al₂O₃, 0 bis 6 Gew.-% B₂O₃, 0 - 8 Gew.-% TiO₂ sowie gegebenenfalls 0 - 2 Gew.-% BaO aufgebracht.

Diese Bonder-Schicht ist insbesondere dann vorteilhaft, wenn eine Haftvermittlung zwischen Keramik-Schicht und Metallkern zur Herstellung einer besseren Haftung nötig ist. Nach dem Brennen der auf den Kern aufgebrachten Bonder-Schicht, wird diese Anordnung dann unter Verwendung der Opaker- und Dentin-/Schmelzmasse wie oben beschrieben weiter verarbeitet.

Durch die Verwendung einer Preopaker-Schicht lassen sich ästhetisch besonders befriedigende Ergebnisse erzielen. Der Preopaker enthält Oxide ausgewählt aus der Gruppe bestehen aus SiO₂, Na₂O, K₂O, Al₂O₃ und ein Reduktionsmittel in Form eines Metalls oder Nichtmetalls ausgewählt aus der Gruppe bestehend aus Sn, Al, Zn, Fe. Vorzugsweise wird eine Preopaker-Schicht zwischen Kern und Opaker-Schicht bzw. zwischen Bonder-Schicht und Opaker-Schicht angeordnet. Dabei wird das jeweils wie beschrieben vorbereitete Material mit einer Preopaker-Masse mit folgender Zusammensetzung 45 bis 55 Gew.-% SiO₂, 4 bis 10 Gew.-% Na₂O, 0 bis 2,5 Gew.-% CaO, 8 bis 14 Gew.-% K₂O, 10 bis 20 Gew.-% Al₂O₃, 0 bis 6 Gew.-% B₂O₃, 0 - 6 Gew.-% TiO₂ und 2 - 20 Gew.-% SnO₂ sowie gegebenenfalls 0 - 2 Gew.-% BaO versehen und nach dem Brennen wie bereits beschrieben weiterverarbeitet.

In einer besonderen Ausführungsform weist der Preopaker eine metallische Komponente, die ein Reduktionsmittel ist, auf. Besonders bevorzugt sind Metalle aus der Gruppe Sn, Al, Zn, Fe, wobei diese einzeln oder im Gemisch enthalten sein können.

Die Reduktionsmittel sind vorzugsweise in Mengen von 1 bis 20 Gew.-%, besonders bevorzugt in Mengen von 1 bis 15 Gew.-% und ganz besonders bevorzugt in Mengen von 5 bis 10 Gew.-%, bezogen auf die Keramikmasse, enthalten. Die Korngröße des Reduktionsmittels kann zwischen 1 und 50 µ liegen. Bevorzugt ist ein Bereich von 1 bis 35µ.

Die jeweiligen Keramikmassen Bonder-, Preopaker-, Opaker- und Dentin-/Schmelzmasse werden vorzugsweise in einer Modellierflüssigkeit auf die jeweils zu verarbeitende Anordnung aufgebracht und dann gebrannt. Die Modellierflüssigkeit, mit der die entsprechenden Massen angemischt werden, basiert vorzugsweise auf wäßrigen Systemen. Die Herstellung des dentalen Metallkeramiksystems erfolgt in an sich bekannter Weise durch Mischen der festen Einzelkomponenten und übliches Auftragen mehrerer Masseschichten auf das Metallgerüst und anschließendes Brennen dieser Massen. Die Keramikmassen werden dabei, wie bereits oben beschrieben, in mehreren Schichten auf das Metallgerüst aufgebracht. Dabei hat es sich bei kupferhaltigen Metallgerüsten als besonders vorteilhaft erwiesen, zunächst auf die Legierung einen sogenannten Bonder aufzubringen, der die Bindung zwischen Metall- und Keramikschicht verbessert, und anschließend eine preopake Schicht aufzubringen, die das Reduktionsmittel enthält, um dann in üblicher Weise die weiteren Schichten, nämlich die Grundmasse, die Dentinmasse, die Schmelzmasse und gegebenenfalls die Effektmasse aufzutragen und nach dem Auftragen der jeweiligen Masse einen Brennvorgang vorzunehmen.

Diese Verfahrensweise hat den Vorteil, daß jetzt auch Legierungen, die bisher nur als Vollgußlegierungen für Metallersatzzähne verwendet wurden, auch für Metallkeramiken verwendet werden können. Weiterhin sind diese Legierungen aufgrund des höheren Kupferanteils erheblich preisgünstiger als die bisherigen Legierungen von Gold und Platin.

Es war überraschend, daß trotz der Zugabe von Reduktionsmitteln, die eigentlich wieder zur Entfernung der Oxidschicht auf der oxidgebrannnten Legierung führen müßten, eine ausreichende Haftung zwischen Metallkomponente und Keramikkomponente erhalten bleibt. Es ist somit gelungen, bisher als nicht verblendbare Vollgußlegierungen geltende kupferhaltige Legierungen auch dem Gebrauch in der Metallkeramik zuzuführen.

Die Wärmeausdehnungskoeffizienten jeder einzelnen Schicht liegen vorzugsweise im Bereich von 14,5 bis 17.5. Die jeweilige Brenntemperatur der zum Einsatz kommenden, die Schichten aufbauenden Massen liegt im Bereich von 700 bis 900°C.

Der Kern einer dentalkeramischen Restauration kann, wie bereits ausgeführt, entweder aus Metall oder selbst aus einer Keramik bestehen. Werden Metallegierungen als Gerüst bzw. Kern verwendet, kann es vorteilhaft sein, zunächst die Bonder-Schicht aufzutragen. In diesem Falle empfiehlt es sich, die Wärmeausdehnungskoeffizienten der Materialien Metallkern und Bonder-Schicht so zu wählen, daß die Differenz des Wärmeausdehnungskoeffizienten im Bereich von 0,5 bis 1,5 Einheiten liegt, wobei die Legierung den höheren Wärmeausdehnungskoeffizienten aufweist. Die darauf nachträglich anzubringenden weiteren Keramikschichten können dann eine weitere Abstufung zu niedrigeren Wärmeausdehnungskoeffizienten erfahren.

Bei Verwendung von Keramikgerüsten oder -kernen ist es ebenfalls vorteilhaft, die Differenz der Wärmeausdehnungskoeffizienten im Bereich von 0,5 bis 1,5 zwischen dem Gerüst oder Kern (Unterbau) und der darauf angeordneten ersten Schicht zu wählen. Je nach der Beschaffenheit des keramischen Kerns kann dabei auch auf das Auftragen einer Bonder-Schicht verzichtet werden.

Gegenstand der vorliegenden Erfindung sind auch die in den Ansprüche 8 bis 12 genannten Zwischenprodukte sowohl als fertige Abmischung in fester, pulveriger Form oder mit entsprechenden Modellierflüssigkeiten angemischt.

Die genannten Komponenten sind geeignet, zur Herstellung der erfindungsgemäßen dentalkeramischen Restauration verwendet zu werden.

Die Erfindung wird an folgendem Beispiel näher erläutert.

### Beispiel

Zunächst wird in bekannter Weise ein Gerüst für die dentalkeramische Restauration (metallischer Kern) ausgearbeitet. Danach wird die Bonder-Masse der Zusammensetzung
57,13 Gew.-% SiO₂, 6,63 Gew.-% Na₂O, 1,47 Gew.-% CaO, 11,47 Gew.-% K₂O, 15,7 Gew.-% Al₂O₃, 3,06 Gew.-% B₂O₃ und 3,7 Gew.-% TiO₂ mit Modellierflüssigkeit angemischt und aufgetragen.

Die Anordnung wird dann bei 800°C im Vakuum gebrannt. Daran schließt sich der Auftrag einer Preopaker-Schicht an. Als Preopaker wird die folgende Mischung verwendet:
51,8 Gew.-% SiO₂, 5,96 Gew.-% Na₂O, 1,27 Gew.-% CaO, 10,02 Gew.-% K₂O, 14,4 Gew.-% Al₂O₃, 2,796 Gew.-% B₂O₃, 3,09 Gew.-% TiO₂, 10,63 Gew.-% SnO.

Nach Auftrag der Preopaker-Schicht wird die Anordnung ebenfalls bei 800°C für eine Zeitdauer von etwa 2 min im Vakuum gebrannt.

Danach wird der Opaker mit der Zusammensetzung
54,8 Gew.-% SiO₂, 7,1 Gew.-% Na₂O, 1,4 Gew.-% CaO, 10,6 Gew.-% K₂O, 15,0 Gew.-% Al₂O₃, 2,3 Gew.-% B₂O₃ und 8,8 Gew.-% TiO₂ mit einer Modellierflüssigkeit angemischt und aufgetragen und bei ca. 800°C gebrannt. Darauf wird dann die Dentin-/Schmelzmasse der folgenden Zusammensetzung 57,5 Gew.-% SiO₂, 6,7 Gew.-% Na₂O, 1,8 Gew.-% CaO, 11,2 Gew.-% K₂O, 17,1 Gew.-% Al₂O₃ und 5,7 Gew.-% B₂O₃ aufgetragen und bei ca. 800°C gebrannt. Daran kann sich ein Glanzbrand unter Verwendung üblicher Malfarben wie Vitachrom "L" und Vitachrom Delta Stains Malfarben anschließen. Dieser Brand wird ebenfalls bei ca. 800°C durchgeführt.

## Patentansprüche

1. Dentalkeramische Restauration mit einem mehrschichtigen Aufbau auf einem Kern, erhältlich durch Aufbringen einer Opaker-Schicht der Zusammensetzung
50 bis 60 Gew.-% SiO₂, 4 bis 10 Gew.-% Na₂O, 0 bis 2,5 Gew.-% CaO, 8 bis 14 Gew.-% K₂O, 10 bis 20 Gew.-% Al₂O₃, 0 bis 6 Gew.-% B₂O₃, 5 bis 12 Gew.-% TiO₂ sowie Pigmenten, vorzugsweise auf Oxidbasis, auf einen Kern, gefolgt von Brennen dieser Anordnung,
danach Aufbringen einer Dentin-/Schmelzschicht der Zusammensetzung 50 bis 60 Gew.-% SiO₂, 4 bis 10 Gew.-% Na₂O, 0 bis 2,5 Gew.-% CaO, 8 bis 14 Gew.-% K₂O, 10 bis 20 Gew.-% Al₂O₃, 2 bis 10 Gew.-% B₂O₃, insbesondere 4 bis 8 Gew.-% B₂O₃ sowie Pigmenten, vorzugsweise auf Oxidbasis, gefolgt von einem Brennen der Anordnung.

2. Dentalkeramische Restauration nach Anspruch 1, wobei als erste Schicht eine Bonder-Schicht mit der folgenden Zusammensetzung
50 bis 60 Gew.-% SiO₂, 4 bis 10 Gew.-% Na₂O, 0 bis 2,5 Gew.-% CaO, 8 bis 14 Gew.-% K₂O, 10 bis 20 Gew.-% Al₂O₃, 0 bis 6 Gew.-% B₂O₃, 0 bis 8 Gew.-% TiO₂ sowie gegebenenfalls 0 - 2 Gew.-% BaO auf den Kern aufgebracht wird und nach Brennen dieser Anordnung die im Anspruch 1 genannten weiteren Schichten aufgebracht werden.

3. Dentalkeramische Restauration nach einem der Ansprüche 1 und/oder 2, wobei zwischen der Opaker-Schicht und dem Kern bzw. der Bonder-Schicht eine Preopaker-Schicht mit der folgenden Zusammensetzung
45 bis 55 Gew.-% SiO₂, 4 bis 10 Gew.-% Na₂O, 0 bis 2,5 Gew.-% CaO, 8 bis 14 Gew.-% K₂O, 10 bis 20 Gew.-% Al₂O₃, 0 bis 6 Gew.-% B₂O₃, 0 - 6 Gew.-% TiO₂ und 2 - 20 Gew.-% SnO₂ sowie gegebenenfalls 0 - 2 Gew.-% BaO aufgebracht wird und nach Brennen dieser Anordnung die Schichten gemäß Anspruch 1 und/oder 2 aufgebracht werden.

4. Dentalkeramische Restauration nach mindestens einem der Ansprüche 1 bis 3, wobei der Wärmeausdehnungskoeffizent jeder einzelnen Schicht im Bereich von 14,5 bis 17,5 und die jeweilige Brenntemperatur im Bereich von 700 bis 900°C liegt.

5. Dentalkeramische Restauration nach mindestens einem der Ansprüche 1 bis 4, wobei der Kern ein metallischer oder keramischer Kern ist.

6. Dentalkeramische Restauration nach mindestens einem der Ansprüche 1 bis 5 mit metallischem oder keramischem Kern, wobei deren Ausdehnungskoeffizienten auf die Keramikschicht abgestimmt sind, daß diese um 0,5 bis 1,5 höher liegen als derjenigen Keramikschicht, die an dem Kern anliegt.

7. Dentalkeramische Restauration gemäß mindestens einem der Ansprüche 1 bis 6, wobei die Metallegierung des metallischen Kerns einen Soliduspunkt von mindestens 50°C oberhalb der Brenntemperatur der Keramikschichten aufweist.

8. Zwischenprodukt in Form eines Bonders mit der Zusammensetzung
50 bis 60 Gew.-% SiO₂, 4 bis 10 Gew.-% Na₂O, 0 bis 2,5 Gew.-% CaO, 8 bis 14 Gew.-% K₂O, 10 bis 20 Gew.-% Al₂O₃, 0 bis 6 Gew.-% B₂O₃, 0 bis 8 Gew.-% TiO₂ sowie gegebenenfalls 0 - 2 Gew.-% BaO.

9. Zwischenprodukt einer Dentalkeramischen Restauration gemäß Anspruch 1 in Form eines Opakers der folgenden Zusammensetzung
50 bis 60 Gew.-% SiO₂, 4 bis 10 Gew.-% Na₂O, 0 bis 2,5 Gew.-% CaO, 8 bis 14 Gew.-% K₂O, 10 bis 20 Gew.-% Al₂O₃, 0 bis 6 Gew.-% B₂O₃, 5 bis 12 Gew.-% TiO₂ sowie Pigmenten, vorzugsweise auf Oxidbasis.

10. Zwischenprodukt einer dentalkeramischen Restauration gemäß Anspruch 1 bis 7 in Form eines Preopakers enthaltend Oxide ausgewählt aus der Gruppe bestehen aus SiO₂, Na₂O, K₂O, Al₂O₃ und ein Reduktionsmittel in Form eines Metalls oder Nichtmetalls ausgewählt aus der Gruppe bestehend aus Sn, Al, Zn, Fe.

11. Zwischenprodukt nach Anspruch 10 mit folgender Zusammensetzung 45 bis 55 Gew.-% SiO₂, 4 bis 10 Gew.-% Na₂O, 0 bis 2,5 Gew.-% CaO, 8 bis 14 Gew.-% K₂O, 10 bis 20 Gew.-% Al₂O₃, 0 bis 6 Gew.-% B₂O₃, 0 - 6 Gew.-% TiO₂ und 2 - 20 Gew.-% SnO sowie gegebenenfalls 0 - 2 Gew.-% BaO und Pigmenten, vorzugsweise auf Oxidbasis.

12. Zwischenprodukt einer dentalkeramischen Restauration gemäß Anspruch 1 in Form einer Dentin-/Schmelzmasse der Zusammensetzung 50 bis 60 Gew.-% SiO₂, 4 bis 10 Gew.-% Na₂O, 0 bis 2,5 Gew.-% CaO, 8 bis 14 Gew.-% K₂O, 10 bis 20 Gew.-% Al₂O₃, 2 bis 10 Gew.-% B₂O₃ sowie Pigmente, vorzugsweise auf Oxidbasis.

13. Verwendung der Zwischenprodukte nach einem der Ansprüche 8 bis 10 zur Herstellung einer dentalkeramischen Restauration nach mindestens einem der Ansprüche 1 bis 7.
